# EUROPEAN PATENT APPLICATION

(11) **EP 2 997 934 A1**
(43) Date of publication of application: **23.03.2016**
(21) Application number: 14798128.6
(22) Date of filing: 10.03.2014
(51) Int. Cl.: A61F 2/16

(54) **ADJUSTABLE INTRAOCULAR LENS**

(30) Priority: 13.05.2013 JP 2013100932
(71) Applicant: Frontier Vision Co., Ltd., Hyogo 663-8111 (JP)
(72) Inventor: AKURA, Junsuke, Higashimuro-gun Wakayama 649-4114 (JP); PKHAREL, Kiran, Nishinomiya-shi Hyogo 663-8111 (JP)
(74) Representative: Viering, Jentschura & Partner mbB Patent- und Rechtsanwälte
(86) International application number: PCT/JP2014/056174
(87) International publication number: WO 2014/185136

(57) **Abstract**

[Abstract] The present invention aims to provide an accommodative intraocular lens capable of sufficiently exerting focusing ability. The present accommodative intraocular lens 1 is provided with an optical section 10 of a lens, and a lens supporting section 20 provided at the peripheral portion of the optical section 10. The lens supporting section 20 includes one end portion 201 connected to the peripheral portion of the optical section 10 via the connecting portion 50 in a movable manner, and the other end portion 202 to be engaged with the equator Se of the lens capsule S. The one end portion 201 and the other end portion 202 are positioned on opposite sides across the center O of the optical section 10, and are formed in a manner as to extend on radially outward side in a loop shape surrounding the periphery of the optical section 10 from one end portion 201 to the other end portion 202. In accordance with the movements of the lens capsule S near the equator Se caused by the focusing of an eye, the other end portions 202 of the lens supporting sections 20 move in the approaching/departing direction, which moves the optical section 10 in the optical axis direction.

## Description

### Technical Field

The present application relates to an accommodative intraocular lens to be inserted into a lens capsule from which contents were removed by cutting an anterior capsule of a lens capsule during cataract surgery, etc.

### Background Technology

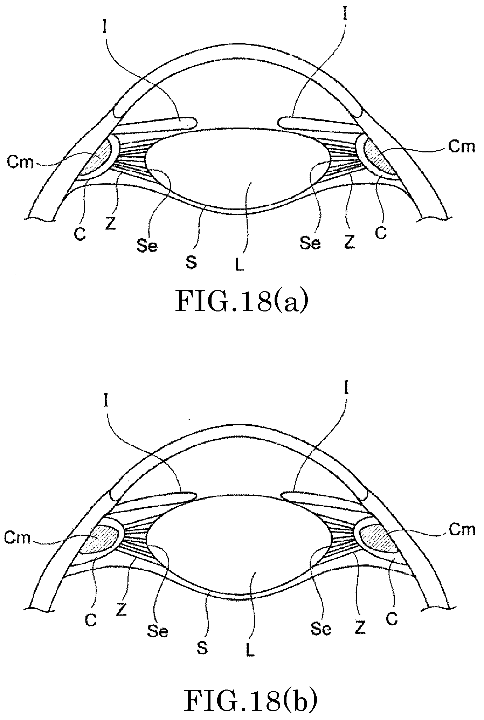

Normally, human eye focusing is performed by changing a thickness of a lens capsule. As shown in Fig. 18, a lens capsule L is a transparent member of a convex shape capable of exerting a lens function, in which a diameter of the lens in the radial direction is about 9 to 10 mm and a thickness of the lens in the fore-and-aft direction is about 4 to 5 mm. The crystalline lens L is fixed to a ciliary body C via Zinn's zonule fibers Z in a manner as to be arranged behind the iris I with the lens encapsulated by the lens capsule S.

Concrete focusing mechanism will be explained as follows. For example, in the case of seeing a far distance, as shown in Fig. 18(a), the ciliary muscle Cm of the ciliary body C is in a relaxed state, and therefore the ciliary body C is in a position retracted in a direction apart from the lens capsule S. In this state, a relatively strong tension is applied to the Zinn's zonule fibers Z positioned between the ciliary body C and the equator Se of the lens capsule. As a result, the equator Se of the lens capsule S is pulled radially outward to cause deformation of the lens so that the thickness of the lens decreases. At the same time, the curvature of the front surface of the crystalline lens L increases, decreasing the refractive powerrefractive power of the crystalline lens L. Thus, focusing is performed at the time of seeing a far distance.

On the other hand, in the case of trying to see a near object, as shown in Fig.18(b), the ciliary muscle Cm of the ciliary body C contracts to cause protrusion of the ciliary body C centripetally (in a direction toward the equator Se of the lens capsule S), which results in a movement of the ciliary body C in a direction approaching the lens capsule S. As a result, the tensile force of the Zinn's zonule fibers X decreases, causing deformation of the crystalline lens so that the thickness of the crystalline lens increases by the elastic force inherent in the crystalline lens L. At the same time, the curvature of the front surface of the crystalline lens L decreases, increasing the refractive power of the crystalline lens L. Thus, focusing is performed at the time of seeing a near distance.

As explained above, in accordance with contraction and relaxation of the ciliary muscle, the ciliary body protrudes or retracts in the radial direction of the lens capsule by a predetermined amount (concretely about 0.3 mm), causing movements of the equator of the lens capsule in the radial direction by the same degree. This causes a change in the thickness of the crystalline lens (concretely by about 0.3 to 0.5 mm). Thus, focusing is performed by deflecting the light entered into an eye. It is known that contraction and relaxation of the ciliary muscle of the ciliary body are kept well even at an old age in the same manner as at a young age. On the other hand, it is also known that contents and a lens capsule of a crystalline lens become hardened to lose the flexibility, resulting in less changes in the thickness of the crystalline lens, which loses the force of voluntary adjusting the focal point (hereinafter referred to as "focusing ability) when seeing a near distance from the state when seeing a far distance (which will be called presbyopia).

By the way, among diseases relating to crystalline lenses, there is a disease called "cataract" in which a crystalline lens becomes cloudy mainly caused by advancing age. Many patients have cataract surgery for treating their cataracts. In the cataract surgery, normally, a method is employed in which a circular hole is formed in the anterior capsule, and the cloudy crystalline lens is removed through the circular hole by an ultrasonic crystalline lens emulsion suction method to remain only a transparent lens capsule in an opened state, and an artificial lens called "intraocular lens" is inserted into the lens capsule. The cataract surgery of this method has been currently applied to patients of more than 1 million in Japan every year and patients of more than 3 million in the United States of America every year.

An intraocular lens generally used for a cataract surgery is provided with an optical section having a refractive power capable of focusing on one of a far distance, an intermediate distance, and a near distance after the surgery depending on the request of the patient, and is called "single focus intraocular lens." This single focal point intraocular lens is fixed to the equator of the lens capsule or therearound by the tensile forces of the lens supporting sections extending from the peripheral portion of the optical section in the radially outward direction. However, since the lens is not designed to change the thickness of the optical section (lens) or move the optical section back and forth like a human eyes crystalline lens and therefore has no focusing ability, it was necessary to compensate the focusing ability by using a pair of eyeglasses having a power corresponding to the distance to an object to be seen.

Further, there is a so-called multifocal intraocular lens in which concentric sections different in refractive power are formed in the lens optical section (this is called "multiple refraction type multifocal intraocular lens") or a structure causing an optical diffraction phenomenon (this is called multiple diffraction type multifocal intraocular lens) is formed in the lens optical section so that light to be entered into an eye is taken in a dispersed manner for a far distance use and a near distance use (in some cases, further for an intermediate distance use). However, in such multifocal intraocular lens, there are reports that patients report a halo phenomenon (an object looks with light rings), a glare (an object looks glistening), insufficient eyesight, or insufficient contrast sensitivity. Thus, such multifocal intraocular lenses could not have sufficiently satisfied patient's requests.

Under the circumstances, there have been conventionally proposed some intraocular lens (accommodative intraocular lens) in which an optical section moves back and forth to perform focusing in accordance with the lens capsule movements accompanied by contract and relaxation of the ciliary muscle of the ciliary body like a human crystalline lens (see the below listed Patent Documents 1 to 3).

The intraocular lens disclosed by Patent Document 1 is generally called "synchrony lens IOL" which is a silicon accommodative intraocular lens in which a convex lens is arranged forward and a concave lens is arranged rearward, both the lenses being connected by spring-like connecting portions.

Further, the accommodative intraocular lens disclosed by Patent Document 2 is generally called "1CU-IOL" which is an acrylic accommodative intraocular lens including one lens and four sheets of movable plate-shaped supporting portion.

Further, the accommodative intraocular lens disclosed by Patent Document 3 is generally called "crystal lens" which is an accommodative intraocular lens made of acryl and polyimide and including one lens, two sheets of plate-shaped members arranged at both peripheral portions of the lens, and a loop-shaped leg formed at the tip end of each plate-shaped member.

### [Prior Art Documents]

### [Patent Document]

[Patent Document ] Japanese Unexamined Patent Application Publication (Translation of PCT Application) No. JP-2005-533611
[Patent Document 2] Japanese Unexamined Patent Application Publication (Translation of PCT Application) No. JP-2004-538086
[Patent Document 3] Japanese Unexamined Patent Application Publication (Translation of PCT Application) No. 2007-512907

### [Summary of the Invention]

### [Problems to be solved by the Invention]

However, it is generally said that a focusing ability required for an accommodative intraocular lens to see from a far distance to a near distance with no problem without glasses is required to be 2.0D or more. A human eye has about 2.0D of focusing ability (pseudoaccommodation) by a focusing ability other than deformation of a lens considered due to a multifocal property, a spherical aberration of a cornea, and a pupillary movement. Adding 2.0D of the pseudoaccommodation and 2.0D of adjustment ability due to the function of the accommodative intraocular lens results in 4.0D, which exerts sufficient adjusting ability. For example, in the case of an accommodative intraocular lens having a normal lens which is +20D in refractive power, a focusing ability of 2.0D or more is exerted when the lens is moved by 0.75 mm in the fore-and-aft direction. Therefore, in order to exert a focusing ability of 2.0D or more, it is required to move the lens by at least 1.5 mm or more.

Considering the above, a conventional accommodative intraocular lens is configured to move the optical section in the fore-and-aft direction utilizing deformation of the lens capsule in accordance with contraction and relaxation by the ciliary muscle of the ciliary body. However, since movement amounts of the ciliary body in the radial direction are small, movement amounts of the equator of the lens capsule are also small (about 0.3 mm). This results in very small movements of the optical section in the fore-and-aft direction. Thus, it is considered to be difficult to exert a focusing ability of 2.0D or more.

Concretely, in Patent Document 1, the equator of the lens capsule moves by about 0.3 mm centripetally, resulting in a forward movement of the equator of the lens capsule by about 0.3 mm centripetally by the focal adjustment. However, even if a lens having a very large refractive power (for example, the refractive power of the lens is +32D) is used as a forward lens and a concave lens having a level of the optical power adjusted to an eye of a patient is used as a rearward lens, only a focusing ability of 0.88D is obtained theoretically. The focusing ability has not reached 2.0D, which cannot exert sufficient focusing ability. Further, the accommodative intraocular lens is an integral member including two sheets of lens and connecting portions connecting the lenses, and therefore the entire volume is rather large. This requires a large incision to insert the accommodative intraocular lens. Thus, it is hard to say that such lens is not preferable as an accommodative intraocular lens.

Further, in Patent Document 2, when the equator of the lens capsule is centripetally moved by about 0.3 mm, it is considered that the lens moves by about 0.6 mm forward. The focusing ability also has not reached 2.0D, which cannot exert sufficient focusing ability. Further, in this accommodative intraocular lens, it is considered that after cataract surgery, etc., the growth of lens epithelial cells occurred near the equator of the lens capsule results in adhesion of the anterior capsule and the posterior capsule and the capsular fibrosis thereof, causing sclerosis of the lens capsule and that the equator of the lens capsule is pressed in the direction of the Zinn's zonule fiber by the tensile force of the lens supporting section in the radially outward direction; as a result, the continuous tonus of the Zinn's zonule fiber has been deteriorated or lost. Therefore, in actual, there is a possibility that the lens does not move forward.

Further, in Patent Document 3, it is configured to move the lens forward at the time of focusing on the assumption that adhesion and capsular fibrosis of the lens capsule occur to a certain degree. However, as to the accommodative intraocular lens, there is no report that the ciliary body protrudes rearward. Further, even if the ciliary body protrudes rearward, it is considered such that when the side of the equator of the lens capsule formed by Zinn's fibers moves rearward, the lens moves rearward. Further, since a movement amount of the ciliary body is small, it is considered that the lens hardly moves. Therefore, it is at least hard to say that sufficient focusing ability is exerted.

As explained above, most of conventional accommodative intraocular lens could not exert sufficient focusing ability. In fact, as for the aforementioned conventional accommodative intraocular lens, many clinical test results have been reported. However, no objective reports demonstrating sufficient movements of the optical section in the fore-and-aft direction, and rather many reports demonstrating no or slight movements of the lens in the fore-and-aft direction have been made. Further, it also is pointed out that focusing effects of the conventional accommodative intraocular lens largely rely on monovision (one of eyes is adjusted to far sight and the other eye is adjusted to near sight to enable seeing from a far object to a near object with both eyes) and/or pseudoaccommodations such as multifocal properties or spherical aberrations of a cornea, or contraction of a pupil.

The present invention was made in view of the aforementioned problems and aims to provide an accommodative intraocular lens capable of sufficiently exerting focusing ability.

### [Means to Solve the Problems]

In order to attain the aforementioned objects, the present invention provides an accommodative intraocular lens to be inserted into a lens capsule from which contents were removed. The accommodative intraocular lens includes: an optical section made of a lens; and a plurality of lens supporting sections formed on a peripheral portion of the optical section. The lens supporting section includes one end portion connected to the peripheral portion of the optical section in a movable manner, and the other end portion to be engaged with an equator of the lens capsule or a lens capsule extension device. The one end portion and the other end portion are positioned on opposite sides across a center of the optical section. The one end portion extends to branch on both sides of the optical section so as to respectively extend along a periphery of the optical section and then are connected with each other to thereby be formed into a loop shape in a manner as to surround the periphery of the optical section as a whole. In accordance with movements of the lens capsule or the lens capsule extension device caused by focusing of an eye, the other end portions of the lens supporting section move in an approaching/departing direction, moving each of one end portions of the lens supporting section in the fore-and-aft direction, which causes movements of the optical section in the fore-and-aft direction in accordance with the movements of the one end portions.

With this, the distance between one end portion and the other end portion of the lens supporting section becomes long, which enables effective conversion of movements of the other end portions of the lens supporting sections in the approaching/departing direction into movements of one end portion of the lens supporting sections in the fore-and-aft direction. For this reason, when the other end portions of the lens supporting sections slightly move in the approaching/departing direction in accordance with the slight movements of the lens capsule or the lens capsule extension device, each one end portion of the lens supporting section moves at the peripheral portion of the optical section, which in turn largely moves each one end portion of the lens supporting section in the fore-and-aft direction. In accordance with the movements, the optical section can be largely moved in the optical axis direction, enabling sufficient exertion of the focusing ability of the accommodative intraocular lens.

Further, the movements of the other end portions of the lens supporting sections in the approaching/departing direction can be assuredly converted into movements of the one end portions in the fore-and-aft direction, which in turn can stably move the optical section in the optical axis direction. At the same time, no symptom such as a glare occurs since there is no obstacle blocking or diffuse light in an optical path passing through the optical section from the pupillary area to the retina.

Further, it is preferable that in the accommodative intraocular lens, the one end portion of the lens supporting section is connected to the peripheral portion of the optical section via a connecting portion in a movable manner. With this, it is possible to simply and assuredly move the one end portion of the lens supporting section by the peripheral portion of the optical section. The one end portion of the lens supporting section or the connecting portion can be provided with a slit extending in a circumferential direction of the optical section. Further, the connecting portion can be provided with one or a plurality of engaging grooves extending in the circumferential direction of the optical section on a rear surface or a front surface of the connecting portion so that one end portion of the lens supporting section is engaged with the engaging groove in a movable manner. The connecting portion also can be provided with one or more engaging hole extending in a circumferential direction of the optical section on a side surface of the connecting portion so that one end portion of the lens supporting section is engaged with the engaging hole in a movable manner.

Further, it is preferable that the lens supporting section includes an urging member for urging an anterior capsule and a posterior capsule of the lens capsule in a manner as to distance the anterior capsule and the posterior capsule in the fore-and-aft direction, the urging member being provided at the other end portion of the lens supporting section. With this structure, the peripheral portion of the equator Se of the lens capsule S is expanded in the fore-and-aft direction to expand the equator Se of the lens capsule S and the equator Se of the lens capsule S is moved centriperally to reduce the diameter of the equator Se of the lens capsule S. This causes continuous tonus of the Zinn's zonule fibers Z. For this reason, the contraction/relaxation of the ciliary muscle is conveyed to the lens capsule via the Zinn' zonule fibers, sufficient focusing ability can be exerted with high dimensional accuracy. Further, the lens capsule equator expands to cause continuous flow of the aqueous humor into the lens capsule equator. This controls the growth or fibrillization of the lens epithelial cell, which makes it possible to control occurrence of after-cataract. Further, the other end portion of the lens supporting section is stably arranged at the vicinity of the equator Se of the lens capsule, which makes it possible to assuredly convey the movements of the lens capsule near the equator to the lens supporting section.

Further, it is preferable that the urging member is formed into one or a plurality of curved shapes. With this structure, it is possible to urge the anterior capsule and the posterior capsule of the lens capsule in a manner as to distance in the fore-and-aft direction with a simple structure.

Further, it is preferable that the urging member is formed into one or a plurality of loop shapes. With this structure, it is possible to more assuredly urge the anterior capsule and the posterior capsule of the lens capsule in a manner as to distance in the fore-and-aft direction.

Further, it can be configured such that the urging member includes an anterior capsule supporting portion supporting the anterior capsule from an inside of the anterior capsule, a posterior capsule supporting portion supporting the posterior capsule from an inside of the posterior capsule, and a connection supporting portion connecting the anterior capsule supporting portion and the posterior capsule supporting portion, and wherein the connection supporting portion urges the anterior capsule supporting portion and the posterior capsule supporting portion in a manner as to distance in the fore-and-aft direction. With this structure, it is possible to more assuredly urge the anterior capsule and the posterior capsule of the lens capsule in a manner as to distance in the fore-and-aft direction.

Further, it is preferable that the urging member is provided at its inner side with an engaging member with which the other end portion of the lens supporting section is engaged. With this structure, the other end portion of the lens supporting section is engaged with the engaging member positioned inward of the connecting portion, and therefore the other end portion of the lens supporting section can be stably arranged near the equator Se of the lens capsule. Further, since the engaging member is not always required to have an urging force unlike the connecting portion, the degree of design freedom of materials, etc., can be enhanced.

Further, it is preferable that the engaging member is formed into a plate shape extending in the fore-and-aft direction, wherein one end portion of the engaging member is connected to the one end portion of the urging member and the other end portion of the engaging member is connected to the other end portion of the urging member, and wherein the engaging member is formed in a manner as to bent toward a direction of the equator of the lens capsule. With this structure, the other end portion of the lens supporting section is engaged with the bent portion of the plate-shaped engaging member, and therefore the other end portion of the lens supporting section can be more stably arranged near the equator of the lens capsule.

Further, it is preferable that a plurality of urging members are arranged along the circumferential direction of the lens capsule, and adjacent urging members are connected in the circumferential direction by two wire members, which constitutes a lens capsule extension device as a whole. With this structure, since urging members are connected in a circumferential direction, each urging member can be stably arranged along the equator of the lens capsule. This enables more stable arrangement of the other end portion of the lens supporting section near the equator of the lens capsule S.

### [Effects of the Invention]

According to the present invention, the distance between one end portion and the other end portion of the lens supporting section becomes long, which enables effective conversion of the movements of the other end portions of the lens supporting sections in the approaching/departing direction into the movements of each one end portion of the lens supporting sections in the fore-and-aft direction. For this reason, when the other end portions of the lens supporting sections slightly move in the approaching/departing direction in accordance with slight movements of the lens capsule or the lens capsule extension device, each one end portion of the lens supporting section largely moves in accordance with the movements at the peripheral portion of the optical section, which in turn largely moves each one end portion of the lens supporting section in the fore-and-aft direction. In accordance with the movements, the optical section can be largely moved in the optical axis direction, enabling sufficient exertion of the focusing ability of the accommodative intraocular lens.

### [Brief Explanation of the Drawings]

[Fig. 1] Fig. 1 is a perspective view illustrating an accommodative intraocular lens according to a first embodiment.
[Fig. 2] Fig. 2 is a plan view of the accommodative intraocular lens of Fig. 1.
[Fig. 3] Fig. 3 is a side view of the accommodative intraocular lens of Fig. 1.
[Fig. 4] Fig. 4(a) to Fig. 4(c) are side views illustrating movements of the accommodative intraocular lens of Fig. 1, and Fig. 4(d) to Fig. 4(f) are side views illustrating movements of a conventional accommodative intraocular lens in a stepwise manner.
[Fig. 5] Fig. 5 is a perspective view illustrating an accommodative intraocular lens provided with an urging member.
[Fig. 6] Fig. 6 shows side views illustrating focusing movements of the accommodative intraocular lens of Fig. 1 implanted in an eye.
[Fig. 7] Fig. 7 is a plan view illustrating a first modification of the accommodative intraocular lens according to the first embodiment.
[Fig. 8] Fig. 8 is a perspective view illustrating a second modification of the accommodative intraocular lens according to the first embodiment.
[Fig. 9] Fig. 9 is a plan view illustrating a third modification of the accommodative intraocular lens according to the first embodiment.
[Fig. 10] Fig. 10 is a side view illustrating an accommodative intraocular lens according to a second embodiment.
[Fig. 11] Fig. 11 is a side view illustrating an accommodative intraocular lens according to a third embodiment.
[Fig. 12] Fig. 12 is a side view illustrating an accommodative intraocular lens according to a fourth embodiment.
[Fig. 13] Fig. 13 is a perspective view illustrating an accommodative intraocular lens according to a fifth embodiment.
[Fig. 14] Fig. 14 illustrates an accommodative intraocular lens according to a sixth embodiment, wherein Fig. 14(a) is a rear view thereof, Figs. 14(b) and 14(c) are side views thereof, and Fig. 14(d) is a cross-sectional view taken along the line B1-B1.
[Fig. 15] Fig. 15 illustrates an accommodative intraocular lens according to a seventh embodiment, wherein Fig. 15(a) is a rear view thereof, Figs. 15(b) and 15(c) are side views thereof, and Fig. 15(d) is a cross-sectional view taken along the line B2-B2.
[Fig. 16] Fig. 16 illustrates an accommodative intraocular lens according to an eight embodiment, wherein Fig. 16(a) is a rear view thereof, Figs. 16(b) and 16(c) are side views thereof, and Fig. 16(d) is a cross-sectional view taken along the line B3-B3.
[Fig. 17] Fig. 7 is a side view illustrating a fourth modification of the accommodative intraocular lens according to the first embodiment.
[Fig. 18] Fig. 18 is a side view illustrating movements of a human eye at the time of focusing.

### [Embodiments For Carrying Out the Invention]

### <First Embodiment>

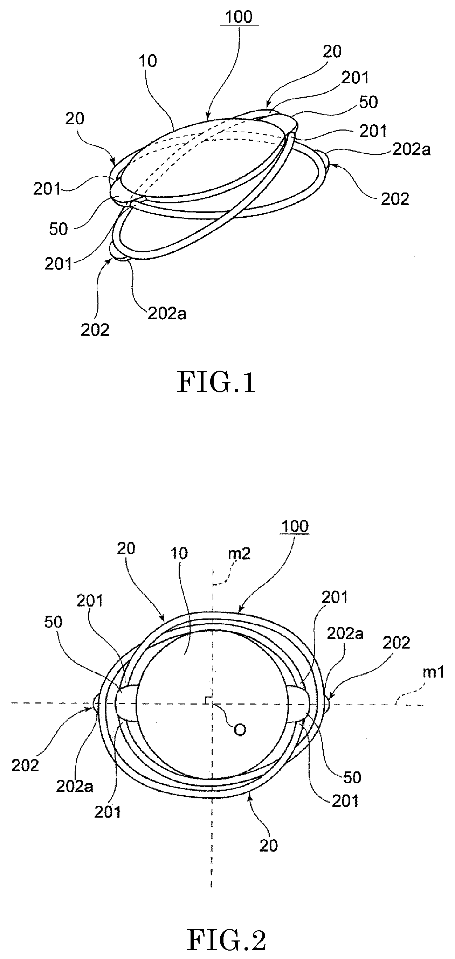

Next, a first embodiment of an accommodative intraocular lens according to the present invention will be explained with reference to Figs. 1 to 6.

### [Structure of Accommodative Intraocular Lens]

The accommodative intraocular lens 100 according to this embodiment is designed to be inserted or implanted into a lens capsule S from which a lens capsule was removed by cataract surgery, etc. As shown in Fig. 1, the accommodative intraocular lens 100 is provided with an optical section 10 of a lens, connecting portions 50 provided at the peripheral portion of the optical section 10, and two lens supporting sections 20 each connected to the peripheral portion of the optical section 10 via the connecting portion 50, the lens supporting sections being the same in size and shape.

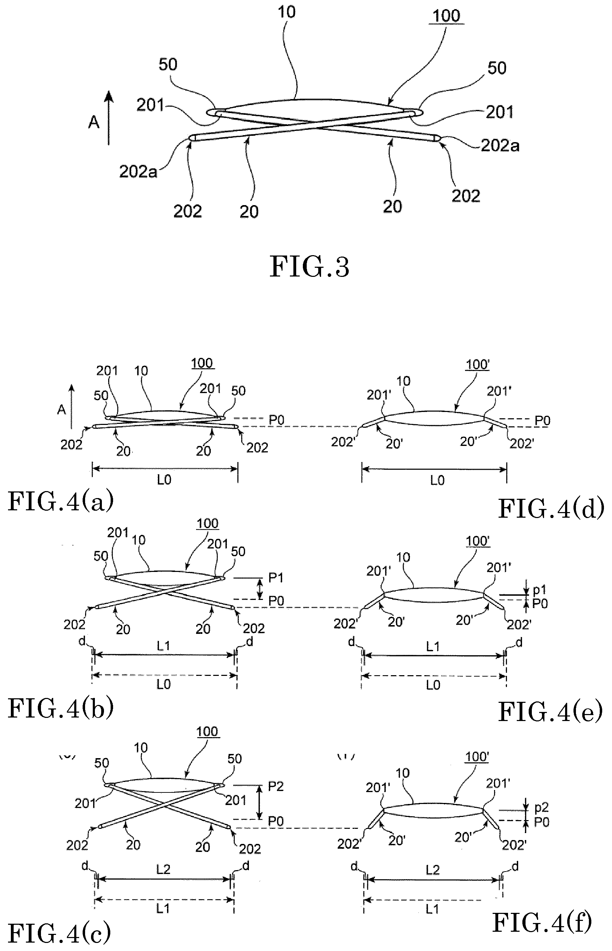

The arrow A shown in Figs. 3 and 4 indicates a forward direction of the accommodative intraocular lens 100 and the opposite direction thereof indicates a rearward direction of the accommodative intraocular lens 100. Further, the fore-and-aft direction of the accommodative intraocular lens 100 means a fore-and-aft direction of an eye which is the same direction as an optical axis direction of the optical section 10.

The optical section 10, as shown in Fig. 1 and Fig. 2, is made of a synthetic material, such as, e.g., silicone, acrylic resin, hydrogel, PMMA, HEMA, hydro polymer, etc., and is provided with a convex lens circular in plan view having a center O.

The connecting portion 50 is a member connecting the optical section 10 and the lens supporting section 20 at the peripheral portion of the optical section 10. The connecting portions 50 are provided so as to protrude from both the right and left side peripheral portions of the optical section 10, and arranged at opposed positions across the center O of the optical section 10.

The lens supporting sections 20, as shown in Fig. 2, are each made of synthetic resin, such as, e.g., PMMA, polyimide, polyvinylidene fluoride, acrylic resin, HEMA, polypropylene, etc., and are arranged symmetrically across the center O of the optical section 10.

Further, each lens supporting section 20 is formed into a loop shape approximately circular in plan view. One end portion 201 of the lens supporting section 20 is connected to the peripheral portion of the optical section 10 via the connecting portion 50 in a movable manner, and the other end portion 202 thereof is configured to be engaged with the equator Se of the lens capsule S or therearound.

Further, in each lens supporting section 20, one end portion 201 and the other end portion 202 are positioned at opposite sides across the center O of the optical section 10. This will be explained concretely as follows. Focusing attention on the lens supporting section 20 having one end portion 201 at the right side in Fig. 2, the straight line connecting one end portion 201 and the other end portion 202 is denoted as "m1," and the straight line passing the center O of the optical section 10 and extending perpendicular to the straight line m1 is denoted as "m2." The other end portion 202 is positioned, among the right and left regions divided by the straight line m2, at the region (the left side region of the straight line m2) opposite to the region where one end portion 201 is positioned (the right side region of the straight line m2). Since one end portion 201 and the other end portion 202 are positioned at opposite sides across the center O of the optical section 10, the distance between the other end portion 202 and one end portion 201 can be kept long.

Further, each lens supporting section 20 is formed in a manner as to extend at the radially outside of the optical section 10. This will be explained concretely as follows. Focusing attention on the lens supporting section 20 having one end portion 201 on the right side of Fig. 2., in the lens supporting section 20, the one end portion 201 extends from the connecting portion 50 while branching on both sides of the optical section 10, each extends along the periphery of the optical section 10 to the other end portion 202, and then both of them join. Thus, the lens supporting section 20 is formed into a loop shape (circular shape) surrounding the periphery of the optical section 10 as a whole. According to this structure, movements of the other end portions 202 of the lens supporting sections 20 in the approaching direction can be assuredly converted into the fore-and-aft directional movements of one end portions 201, which enables stable movements of the optical section 10 in the optical axis direction. Further, it becomes possible that the lens supporting sections 20 can be arranged so as not to cross the optical section 10.

Further, each lens supporting section 20 is configured such that the lens supporting sections 20 do not come into contact with each other when the optical section 10 moves in the optical axis direction. Explaining concretely, as shown in Fig. 2, at the portion where the lens supporting section 20 having one end portion 201 at the right side in Fig. 2 and the lens supporting section 20 having one end portion 201 at the left side in Fig. 2 cross, both the lens supporting sections are arranged so as to be shifted in a plane direction.

Further, in each lens supporting section 20, as shown in Fig. 3, the other end portion 202 is positioned more rearward of the optical section 10 than one end portion 201. For this reason, when both the other end portions 202 move in an approaching/departing direction, the movements of the other end portions 202 in the radial direction are smoothly converted into movements of one end portions 201 in the fore-and-aft direction. This enables assured movements of the optical section 10 in the optical axis direction.

Further, in each lens supporting section 20, at least one end portion 201 is made of an elastic member having a predetermined restoring force, so that the connection to the optical section 10 is in a movable state with the restoring force of the resilient member. This will be explained concretely as follows. As shown in Fig. 4(a), each lens supporting section 20 is normally in a state in which the lens supporting section 20 and the optical section 10 are positioned approximately in the same plane direction. At this state, no restoring force is acted to one end portion. On the other hand, as shown in Figs. 4(b) and 4(c), when the other end portions 202 move in the approaching direction and therefore the one end portions 201 move forward, a restoring force for pulling backward to the state shown in Fig. 4(a) acts on one end portions 201.

Further, as shown in Figs. 1 and 2, each lens supporting section 20 is provided with a connecting ledge 202a protruded from the other end portion 202 radially outward, so that an urging member 30, which will be explained later, can be secured to the other end portion 202 by inserting the connecting ledge 202a into the urging member 30.

Thus, in the accommodative intraocular lens 100 structured as explained above, when an external force is applied to each of the other end portions 202 of each lens supporting section 20 in the radially inward direction, the other end portions 202 move in the approaching direction. This results in a forward movement of the optical section 10. On the other hand, when the external force applied to each of the other end portions 202 of the lens supporting section 20 in the radially inward direction of the optical section 10 is released, the other end portions 202 move in the departing direction by the action of the restoring forces of one end portions 201. This results in a rearward movement of the optical section 10 so that the optical section 10 returns to its original position.

Hereinafter, the mechanism of movements of the optical section 10 in the optical axis direction will be concretely explained with reference to Figs. 4(a) to 4(c).

Initially, in this accommodative intraocular lens 100, as shown in Fig. 4(a), each lens supporting section 20 is positioned approximately in the plane direction of the optical section 10. At this time, the position of the optical section 10 in the fore-and-aft direction is denoted as a reference position P0, and the distance between the other end portions 202 is denoted as L0.

Then, in this accommodative intraocular lens 100, as shown in Fig. 4(b), when external forces are applied radially inward of the optical section 10 to the other end portions 202 of the lens supporting sections 20 to move the other end portions 202 by a movement amount d in the approaching direction and the distance between the other end portions 202 becomes L1, the posture of each lens supporting section 20 gradually changes from a state parallel to the plane direction to a state inclined in the fore-and-aft direction. This is because the force applied to the other end portion 202 in the radial direction of the optical section 10 is converted into a force in the fore-and-aft direction of one end portion 201 based on the structure in which the lens supporting sections 20 are symmetrical with respect to the optical section 10 and the structure in which the other end portion 202 of the lens supporting section 20 is positioned more rearward than one end portion 201. With this, one end portion 201 moves forward to the position P1, which in turn can move the optical section 10 connected to the one end portions 201 of both the lens supporting sections 20 via the connecting portions 50 forward in the optical axis direction to the position P1.

Further, in this accommodative intraocular lens 100, as shown in Fig. 4(c), when external forces are applied to the other end portions 202 of lens supporting sections 20 toward the radially inward direction of the optical section 10 to further move the other end portions 202 by a movement amount d in the approaching direction and the distance between the other end portions 202 becomes L2, one end portions 201 further move forward to the position P2. In accordance with the movement, the optical section 10 also moves forward in the optical axis direction to the position P2.

On the other hand, in the accommodative intraocular lens 100 in the state shown in Fig. 4(c), as shown in Fig. 4(b), when the external forces applied to the other end portions 202 of the lens supporting section 20 in the radially inward direction of the optical section 10 are released to move the other end portions 202 by a movement amount d in the departing direction by the function of the restoring force of each one end portion 201 and the distance between the other end portions 202 becomes L2 to L1, each lens supporting section 20 deforms in a manner such that the posture thereof changes from the fore-and-aft direction to the plane direction. With this, one end portion 201 moves rearward to the position P1. In accordance with this movement, the optical section 10 connected to one end portion 201 of the lens supporting section 20 via the connecting portion 50 also can move rearward in the optical axis direction to the position P1.

In the accommodative intraocular lens 100, as shown in Fig. 4(a), when each of the other end portions 202 further moves in the departing direction by a movement amount d and the distance between the other end portions 202 changes from L1 to L0, the one end portion 201 further moves rearward to the position P0. In accordance with the movement, the optical section 10 further moves rearward in the optical axis direction to the position P0 to be returned to its original state.

In the aforementioned accommodative intraocular lens 100, comparing to a conventionally known accommodative intraocular lens (for example, the accommodative intraocular lens shown in Patent Document 2, hereinafter referred to as "conventional accommodative intraocular lens") 100', it becomes possible to effectively convert movements of the other end portions 202 of the lens supporting section 20 in the approaching/departing direction into movements of the optical section 10 in the fore-and-aft direction.

Hereinafter, this will be explained by comparing the accommodative intraocular lens 100 and the conventional accommodative intraocular lens 100'. Both the accommodative intraocular lens 100 and the conventional accommodative intraocular lens 100' are provided with the same optical section 10 and allotted by the same number to the corresponding structure, and both the intraocular lens are distinguished by allotting a dash (') to the numerals of the conventional accommodative intraocular lens. Further, the comparison is made under the condition that the movement of the other end portion is the same movement amount d.

In the lens supporting section 20 of the accommodative intraocular lens 100, as shown in Fig. 4(a), the other end portion 202 is positioned at the region opposite to the region in which one end portion 201 is positioned. On the other hand, in the lens supporting section 20' of the conventional intraocular lens 100', as shown in Fig. 4(d), both of one end portion 201' and the other end portion 202' are positioned on the same side. For this reason, the distance from one end portion 201 to the other end portion 202 in the accommodative intraocular lens 100 is longer than the distance from one end portion 201' to the other end portion 202' of the conventional intraocular lens 100'.

Considering the above, in the conventional accommodative intraocular lens 100', as shown in Fig. 4(d), each lens supporting section 20' is arranged approximately in the plane direction of the optical section 10. The position of the optical section 10 in the fore-and-aft direction at this time is a reference position P0, and the distance between the other end portions 202 of the lens supporting sections 20 is L0.

In the conventional accommodative intraocular lens 100', as shown in Fig. 4(e), when external forces are applied to the other end portions 202' of the lens supporting sections 20' in the radially inward direction to move the other end portions 202' in the approaching direction by a movement amount d and the distance between the other end portions 202' becomes L1, each lens supporting section 20' deforms in a manner such that the posture thereof gradually changes from the plane direction to the fore-and-aft direction. However, in the conventional accommodative intraocular lens 100', since the distance from one end portion 201' to the other end portion 202' is shorter than in the accommodative intraocular lens 100, the one end portion 201' only moves to the position p1. As will be apparent by comparing Fig. 4(b) and Fig. 4(e), the position p1 in the conventional accommodative intraocular lens 100' is positioned more rearward than the position P1 in the present accommodative intraocular lens 100.

In the conventional accommodative intraocular lens 100', as shown in Fig. 4(f), when external forces are further applied to the other end portions 202' of the lens supporting sections 20' in the radially inward direction to further move each of the other end portions 202' in the approaching direction by a movement amount d and the distance between the other end portions 202' becomes L2, one end portion 201' further moves forward to the position p2. In accordance with the movement, the optical section 10 also further moves forward to the position p2. However, in the conventional accommodative intraocular lens 100', since the distance from one end portion 201' to the other end portion 202' is shorter than in the present accommodative intraocular lens 100, as will be apparent from the comparison between Fig. 3(c) and Fig. 3(f), the position p2 of the conventional accommodative intraocular lens 100' is more rearward than the position P2 of the present accommodative intraocular lens 100.

As explained above, in the present accommodative intraocular lens 100, the distance from one end portion 201 to the other end portion 202 is longer than in the conventional accommodative intraocular lens 100'. Therefore, even in cases where the other end portion 202 moves by the same movement amount in the same manner as in the conventional accommodative intraocular lens 100', the movements of the other end portions 202 of the lens supporting sections 20 in the approaching/departing direction can be effectively converted into movements of one end portions 201 of the lens supporting sections 20 in the fore-and-aft direction. As a result, the movements can be effectively converted into movements of the optical section 10 in the fore-and-aft direction.

For this reason, when the other end portions 202 of the lens supporting sections 20 slightly move in the approaching/departing direction in accordance with the slight centripetal and centrifugal (i.e., radially inward and radially outward) movements of the lens capsule S in the vicinity of the equator Se thereof, each one end portion 201 and/or connecting portion 50 of the lens supporting section elastically deforms. This moves one end portion 201 of the lens supporting section 20 at the peripheral portion of the optical section 10. As a result, each end portion 201 of the lens supporting section 20 largely moves in the fore-and-aft direction, resulting in large movements of the optical section 10, which makes it possible to sufficiently exert the focusing ability.

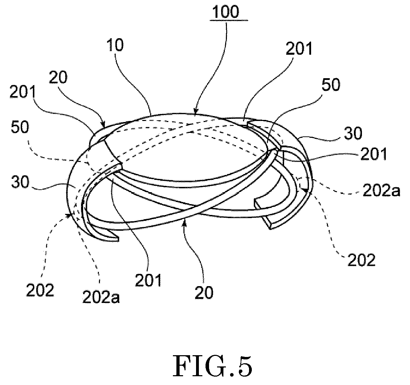

In the aforementioned accommodative intraocular lens 100, as shown in Fig. 5, it is preferable that the lens supporting section 20 is provided with one urging member (hereinafter referred to as "urging member") at the other end portion 202. This urging member 30 is formed into a curved shape in a manner as to expand radially outward from the upper end portion to the lower end portion, and made of a synthetic resin elastic material, such as, e.g., silicon, acrylic, HEMA, hydrogel, PMMA, polyimide, polyvinylidene fluoride, or polypropylene. This urging member 30 is provided with a connection hole, not illustrated, for allowing an insertion of the connecting ledge 202a of the other end portion 202 to fix the other end portion 202 at the inner side lower end section of the urging member 30. Therefore, by inserting the connecting ledge 202a into the connection hole 202a, the urging member 30 is fixed to the other end portion 202.

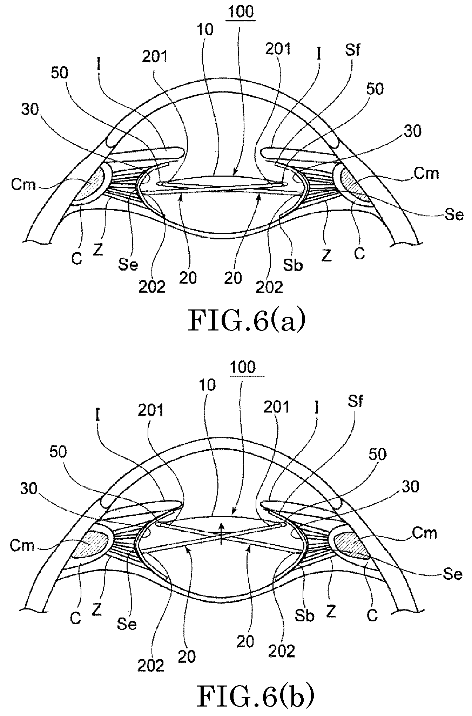

As shown in Fig. 6, when the present accommodative intraocular lens 100 is inserted or implanted into an eye in such a manner that the urging member 30 is in contact with the peripheral portion of the equator Se of the lens capsule S, the anterior capsule Sf and the posterior capsule Sb of the lens capsule S can be urged to distance with each other. For this reason, the peripheral portion of the equator Se of the lens capsule S is expanded in the fore-and-aft direction to expand the equator Se of the lens capsule S and the equator Se of the lens capsule S moves centripetally to reduce the diameter of the equator Se of the lens capsule S, resulting in a consecutive stress of the Zinn's zonule fibers Z. As a result, the contraction and relaxation of the ciliary muscle Cm can be conveyed to the lens capsule S via the Zinn's zonule fibers Z. Further, since the urging member 30 is formed into a curved shape, the anterior capsule Sf and the posterior capsule Sb of the lens capsule S can be urged so as to distance in the fore-and-aft direction with a simple structure.

Further, the equator Se of the lens capsule S expands to allow the continuous flow of the aqueous humor into the equator Se of the lens capsule S to wash away mediators, such as, e.g., cytokine created by the lens epithelial cell, by itself advancing the growth of the lens epithelial cell. This controls growth and/or fibrillization of the lens epithelial cell, which makes it possible to control occurrence of after-cataract. Further, the other end portion 202 of the lens supporting section 20 is stably arranged at the vicinity of the equator Se of the lens capsule S, which makes it possible to assuredly convey the movements of the lens capsule S near the equator Se to the lens supporting section 20.

### [Arrangement and Function of Present Accommodative Intraocular Lens]

Next, the arrangement and function of the present accommodative intraocular lens 100 will be explained with reference to Fig. 6.

In arranging the present accommodative intraocular lens 100, as shown in Fig. 6(a), the anterior capsule Sf of the lens capsule S is incised during cataract surgery, and an accommodative intraocular lens 100 is inserted into the lens capsule S from which contents were removed. At this time, the urging member 30 is brought into contact with the peripheral portion of the equator Se of the lens capsule S. As a result, the peripheral portion of the equator Se of the lens capsule S is expanded in the fore-and-aft direction and the equator Se of the lens capsule S is moved centripetally to reduce the diameter of the equator Se of the lens capsule S. This causes appropriate continuous tonus of the Zinn's zonule fibers Z. Thus, the contraction and relaxation of the ciliary muscle Cm of the ciliary body C can be conveyed to the lens capsule S.

Next, the function of the present accommodative intraocular lens 100 inserted or implanted in an eye will be explained. As shown in Fig. 6(a), at the time of seeing a far distance (at the time of not focusing), the ciliary muscle Cm of the ciliary body C is relaxed and becomes in a flat shape, and the ciliary body C is in a state in which the ciliary body C is retracted toward the radially outside. For this reason, the stress applied to the Zinn's zonule fibers Z increases, so that the equator Se of the lens capsule S and vicinity thereof are pulled radially outward. This results in a state in which the distance between the anterior capsule Sf and the posterior capsule Sb near the equator Se is small. In accordance with this, the urging member 30 is shrank in the fore-and-direction, causing radially outward movements of the other end portions 202 of the lens supporting sections 20. With this, the other end portions 202 move in the departing direction by the resilient forces of both the lens supporting sections 20 to increase the distance between the other end portions 202, causing each of the one end portions 201 to be positioned slightly more forward than each of the other end portions 202. In accordance with this, the optical section 10 is positioned at the center or the rearward side of the lens capsule. As explained above, by positioning the optical section 10 to the rearward side in the optical axis center depending on the position of the surface of the ciliary body C due to the relaxation of the ciliary muscle Cm, the focusing ability can be attained at the time of seeing a far distance.

On the other hand, as shown in Fig. 6(b), at the time of seeing a near distance (at the time of focusing), the ciliary muscle Cm of the ciliary body C contracts, resulting in a centripetally protruded state (toward the lens capsule S side). For this reason, the stress applied to the Zinn's zonule fibers Z weakens, reducing the stress applied to the equator Se of the lens capsule S and therearound, which causes an expansion of the urging member in the fore-and-aft direction and a forward movement of the lens capsule S. This causes a radially inward movement of the other end portion 202 of the lens supporting section 20. With this, when the other end portions 202 engaged with the peripheral portion of the equator Se of the lens capsule S move in the approaching direction to reduce the distance between the other end portions 202, each one end portion 201 is positioned more forward than the other end positon 202. In accordance with this movement, the optical section 10 is positioned more forward in the optical axis direction. As explained above, since the optical section 10 can be positioned forward depending on the movement of the surface of the ciliary body C due to the contraction of the ciliary muscle Cm, the focusing ability at the time of seeing near distance can be exerted.

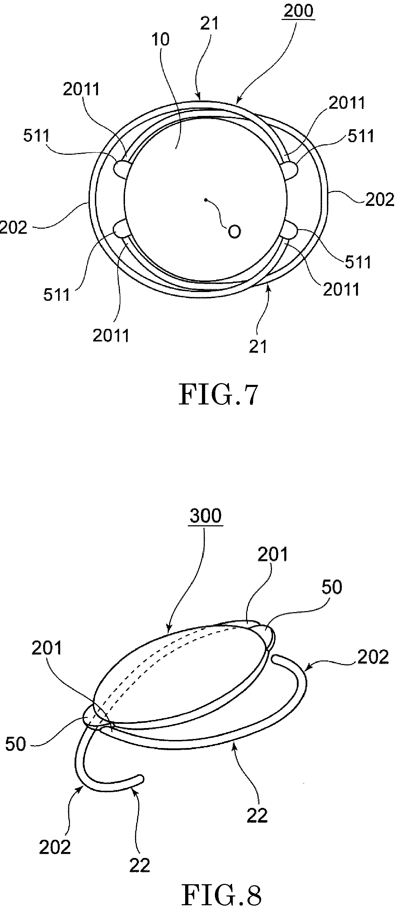

In this embodiment, the lens supporting section 20 having the structure as shown in Fig. 2 is employed, however, other lens supporting sections having other structures can be employed. For example, in the lens supporting section 21 of the accommodative intraocular lens 200 shown in Fig. 7, one of the lens supporting sections 21 having one end portions 2011 on the right side of Fig. 7 is formed in a manner as to extend outside the other of the lens supporting sections 21 having one end portions 2011 on the left side of Fig. 7 at the crossing portion of both the lens supporting sections 21. Alternatively, to the contrary, the lens supporting section 21 having one end portions 2011 on the left side of Fig. 7 can be formed in a manner as to extend outside the lens supporting section 21 having one end portions 2011 on the right side of Fig. 7.

Further, as shown in Fig. 7, it can be configured such that the connecting portion 51 connecting one lens supporting section 21 to the optical section 10 includes two connecting portions 511 and 511 in the circumferential direction and the one end portions 2011 and 2011 of the lens supporting section 21 are respectively connected to these two connecting portions 511 and 511 so as to be connected to the peripheral portion of the optical section 10 at two portions. Concretely, the lens supporting section 21 extends from the connecting portions 511 and 511 on both sides of the optical section 10 so as to respectively extend along the periphery of the optical section 10 and then are connected with each other to thereby formed into a loop shape (circular shape) in a manner as to surround the periphery of the optical section 10 as a whole. In this modification, although the lens supporting section 21 is not formed into a complete circular or loop shape since both one end portions 2011 and 2011 are detached, even in cases where one end portions 2011 and 2011 are detached as mentioned above, in the present invention, such a structure is defined as a loop shape. Further, even in cases where the other end portions 202 are separated, as long as it is structured so as to surround the periphery of the optical section 10 in the same manner, in the present invention, such a structure is also defied as a loop shape.

Further, although the lens supporting section 20 is formed into an approximately-circular loop shape, the lens supporting section 20 can be formed into other shapes. For example, as shown in Fig. 8, in the accommodative intraocular lens 300, it can be configured such that each lens supporting section 22 is formed into an approximately-loop shape extending on an arched line from one end portion 201 to the other end portion 202, the one end portion 201 is connected to the peripheral portion of the optical section 10 in a movable manner, the other end portion 202 is engaged with the equator Se of the lens capsule S or the lens capsule device, and one end portion 201 and the other end portion 202 are positioned at opposite sides across the center O of the optical section 10.

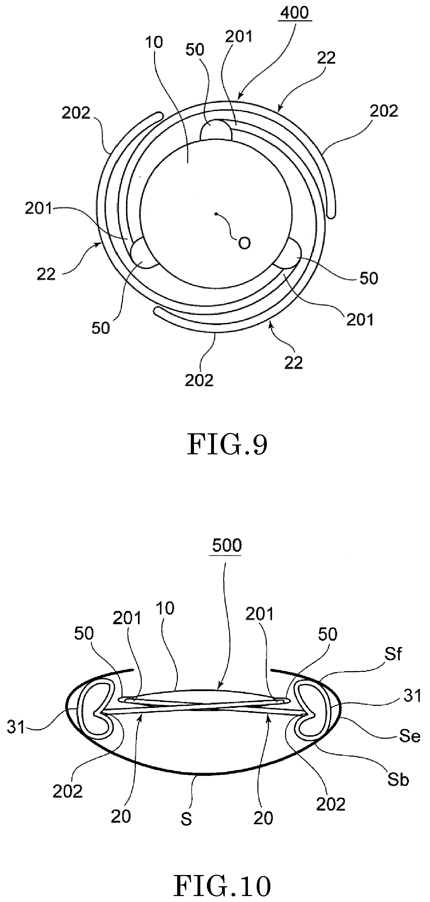

Further, although two lens supporting sections 22 are provided, as shown in Fig. 9 for example, in the intraocular lens 4, three, four or more lens supporting sections 22 can be provided at the peripheral portion of the optical section 10.

Further, although at least one end portion 201 of the lens supporting section 20 is made of an elastic member having a restoring force, the lens supporting section 20 is not required to have a restoring force as long as the one end portion 201 is connected to the peripheral portion of the optical section 10 in a movable manner, and the material is not limited to an elastic member.

Further, although one end portion 201 of the lens supporting section 20 is connected to the peripheral portion of the optical section 10 via the connecting portion 50, the one end portion 201 can be directly connected to the peripheral portion of the optical section 10.

Next, second to eight embodiments according to the accommodative intraocular lens of the present invention will be explained with reference to Figs. 10 to 17. In the following explanation, only the structure different from the structure of the aforementioned embodiment will be explained, and the explanation of the same structure will be omitted by allotting the same symbol.

### <Second Embodiment>

In an accommodative intraocular lens 500 of this embodiment, as shown in Fig. 10, each lens supporting section 20 is provided with an urging member 31 formed into a loop shape as seen from the side at the other end portion 202.

According to this urging member 31, it is possible to more assuredly urge the anterior capsule Sf and the posterior capsule Sb of the lens capsule S in a manner as to distance in the fore-and-aft direction. Further, it is possible to consecutively apply a stress to the Zinn's zonule fibers Z and make the aqueous humor flow into the equator Se of the lens capsule S, and therefore occurrence of after-cataract can be prevented. Further, since the urging member 31 functions as a cushion member between the equator Se of the lens capsule S and the other end portion 202 of the lens supporting section 20, it is possible to reduce the force for reducing the stress of the Zinn's zonule fibers Z by radially outwardly expanding the equator Se of the lens capsule S.

### <Third Embodiment>

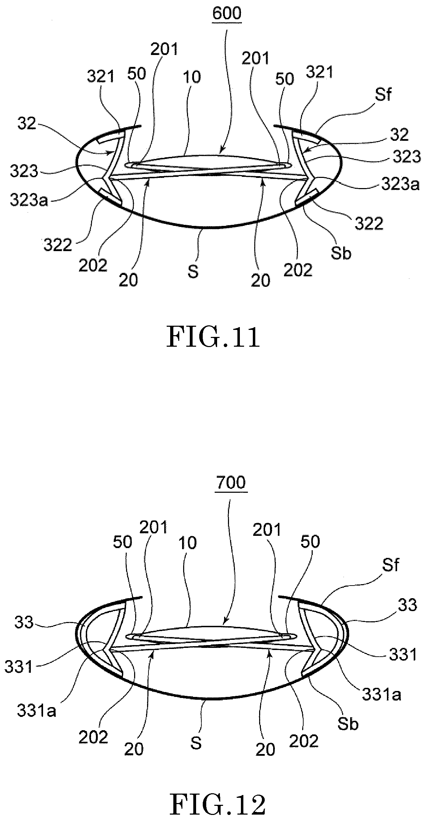

Next, a third embodiment of an accommodative intraocular lens according to the present invention will be explained with reference to Fig. 11.

In the intraocular lens 600 of this embodiment, the urging member 32 is provided with an anterior capsule supporting portion 321 configured to support the anterior capsule Sf from the inside thereof, a posterior capsule supporting portion 322 configured to support the posterior capsule Sb from the inside thereof, and a connection supporting portion 323 connecting the anterior capsule supporting portion 321 and the posterior capsule supporting portion 322 and having a bent portion 323a capable of being bent radially outward. The connection supporting portion 323 urges the anterior capsule supporting portion 321 and the posterior capsule supporting portion 322 in a manner as to distance in the fore-and-aft direction. Further, the other end portion 202 of the lens supporting section 20 is engaged with the bent portion 323a of the urging member 32.

According to this urging member 32, accompanying the opening movement of the anterior capsule Sf and the posterior capsule Sb in the fore-and-aft direction by the near accommodation, when the anterior capsule supporting portion 321 and the posterior capsule supporting portion 322 move in the departing direction and therefore the bending degree of the bent portion 323a of the connection supporting portion 323 in the direction of the equator Se (radially outward) decreases, the other end portions 202 of the lens supporting sections 20 move in the approaching direction. In accordance with the movement, the optical section 10 can be moved forward in the optical axis direction.

### <Fourth Embodiment>

Next, a fourth embodiment according to the accommodative intraocular lens of the present invention will be explained with reference to Fig. 12.

In the accommodative intraocular lens 700 of this embodiment, the urging member 33 is formed by a curved plate formed into a curved shape in a manner as to expand radially outward from the upper end to the lower end. Further, this urging member 33 is provided at tis inner side with an engaging member 331 with which the other end portion 202 of the lens supporting section 20 is engaged.

This engaging member 331 is formed into a plate shape extending in the fore-and-aft direction. One end portion of the engaging member 331 positioned at the forward side is connected to the forward side one end portion of the urging member 33, and the other end portion of the engaging member 331 positioned at the rearward side is connected to the other end portion of the urging member 33. The engaging member 331 includes a bent portion 331a bent in the direction of the equator S2 of the lens capsule S (radially outward).

According to this urging member 33, the other end portion 202 of the lens supporting section 20 is engaged with the engaging member 331 positioned inward of the urging member 33, and therefore the other end portion 202 of the lens supporting section 20 can be stably arranged near the equator Se of the lens capsule S. Further, the other end portion 202 of the lens supporting section 20 is engaged with the bent portion 331a of the plate-shaped engaging member 331, and therefore the other end portion 202 of the lens supporting section 20 can be more stably arranged near the equator Se of the lens capsule S.

### <Fifth Embodiment>

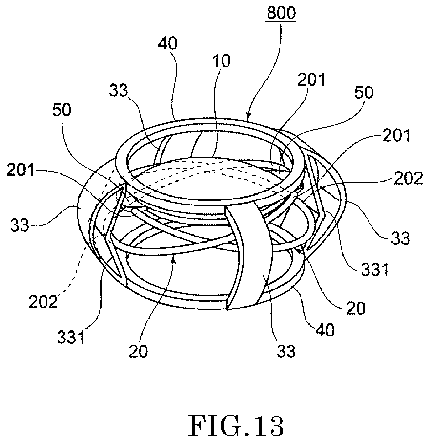

Next, a fifth embodiment according to the accommodative intraocular lens of the present invention will be explained with reference to Fig. 13.

In the accommodative intraocular lens 800 of this embodiment, a plurality of urging members 33 are arranged along the circumferential direction of the lens capsule S, and adjacent urging members 33 are connected in the circumferential direction by two wire members 40, which constitutes a lens capsule extension device as a whole.

According to this structure, since urging members 33 are connected in the circumferential direction by each wire member 40, each urging member 33 can be stably arranged along the equator Se of the lens capsule S. This enables more stable arrangement of the other end portion 202 of the lens supporting section 20 near the equator Se of the lens capsule S.

### <Sixth Embodiment>

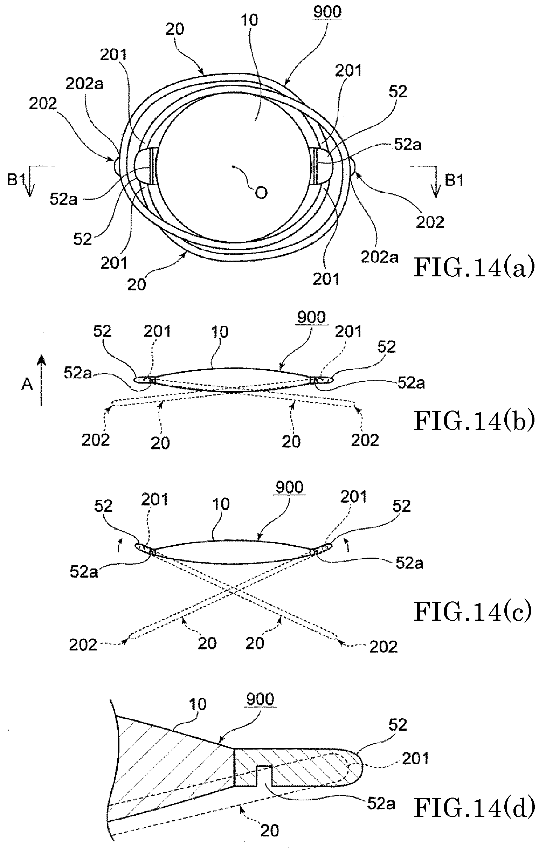

Next, a sixth embodiment according to the accommodative intraocular lens of the present invention will be explained with reference to Fig. 14.

In the accommodative intraocular lens 900 of this embodiment, as shown in Figs. 14(a) and 14(d), the connecting portion 52 is provided with a slit 52a extending in the circumferential direction of the optical section 10 at the position between one end portion 201 of the lens supporting section 20 and the optical section 10 on the rear surface. With this, in accordance with the movement of the other end portions 202 of the lens supporting sections 20 in the approaching direction, when the optical section 10 moves forward via one end portion 201, as shown in Fig. 14(b) and 14(c), the one end portion 201 side of the connecting portion 52 is bent forward. This simply and assuredly moves the one end portion 201 of the lens supporting section 20 by the peripheral portion of the optical section 10.

The slit 52a is formed on the rear surface of the connecting portion 52. However, the slit can be formed on the front surface of the connecting portion 52 so that the one end portion 201 of the lens supporting section 20 can be bent rearward via the slit 52a when the optical section 10 moves forward. Further, such a slit can be formed in one end portion 201 of the lens supporting section 20 so that the one end portion 201 of the lens supporting section 20 can be bent via the slit when the optical section 10 moves forward.

### <Seventh Embodiment>

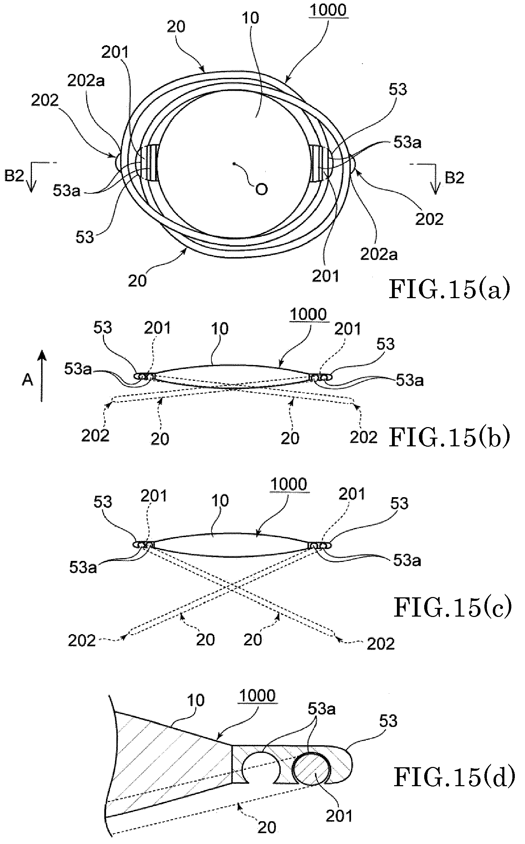

Next, a seventh embodiment according to the accommodative intraocular lens of the present invention will be explained with reference to Fig. 15.

In this accommodative intraocular lens 1000 according to this embodiment, the connecting portion 53 has, as shown in Figs. 15(a) and 15(d), two engaging grooves 53a extending in the circumferential direction of the optical section 10. One end portion 201 of the lens supporting section 20 is rotatably engaged with the outer engaging groove 53a from the rear side. With this structure, in accordance with the movement of the other end portions 202 of the lens supporting sections 20 in the approaching direction, when the optical section 10 moves forward, as shown in Figs. 15(b) and 15(c), one end portion 201 of the lens supporting section 20 rotatably moves in the engaging groove 53a. This easily and assuredly moves the one end portion 201 of the lens supporting section 20 at the peripheral portion of the optical section 10.

Although the one end portion 201 of the lens supporting section 20 is engaged with the outer engaging groove 53a, it can be configured such that the one end portion is engaged with the inner engaging groove 53a.

Since the engaging groove 53a is formed so as to be detached from and engaged with one end portion 201 of the lens supporting section 20, when the position of the accommodative intraocular lens 1000 in the fore-and-aft direction is not appropriate during the surgery or when there is a slight error in the power of the optical section 10 predicted before the surgery, the position of the accommodative intraocular lens 1000 can be corrected by detaching the lens supporting section 20 from the optical section 10 and engaging the lens supporting section 20 again while shifting the position, or the optical section 10 can be detached from the lens supporting section 20 to replace the optical section 10 with a new optical section 10 having a correct power.

Although two engaging grooves 53a are formed, one or three or more engaging grooves can be formed.

Although the engaging groove 53a is formed on the rear surface of the connecting portion 53, the engaging groove can be formed on the front surface of the connecting portion 53.

### <Eighth Embodiment>

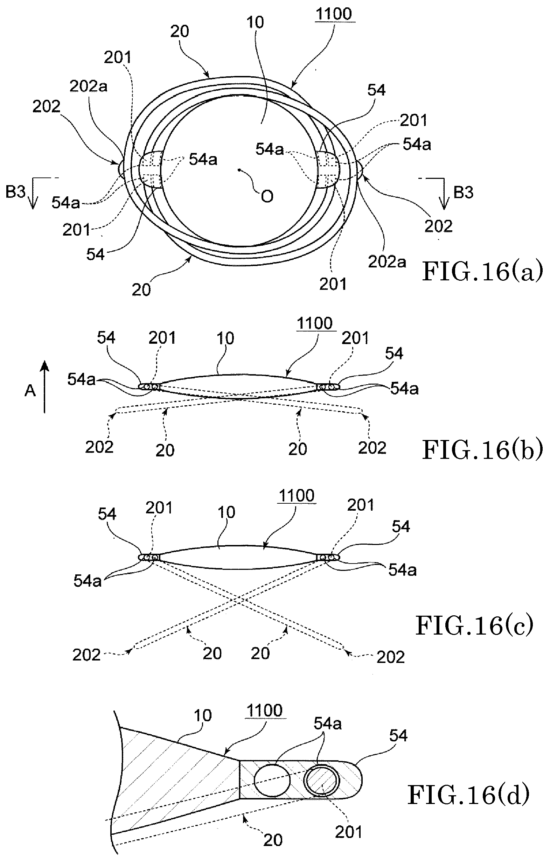

Next, an eighth embodiment according to the accommodative intraocular lens of the present invention will be explained with reference to Fig. 16.

In this accommodative intraocular lens 1100 according to this embodiment, the connecting portion 54 has, as shown in Figs. 16(a) and 16(d), two engaging holes 54a extending in the circumferential direction of the optical section 10 on both side surfaces. One end portion 201 of the lens supporting section 20 is rotatably engaged with the outer engaging hole 54a from the side. With this structure, in accordance with the movement of the other end portions 202 of the lens supporting sections 20 in the approaching direction, when the optical section 10 moves forward, as shown in Figs. 16(b) and 16(c), one end portion 201 of the lens supporting section 20 rotatably moves in the engaging hole 54a. This enables easy and assured movements of the one end portion 201 of the lens supporting section 20 at the peripheral portion of the optical section 10.

Although the one end portion 201 of the lens supporting section 20 is engaged with the outer engaging hole 54a, it can be configured such that the one end portion is engaged with the inner engaging hole 54a.

Since the engaging hole 54a is formed so as to be detached from and engaged with one end portion 201 of the lens supporting section 20, when the position of the accommodative intraocular lens 1100 in the fore-and-aft direction is not appropriate during the surgery or when there is a slight error in the power of the optical section 10 predicted before the surgery, the position of the accommodative intraocular lens 1100 can be corrected by detaching the lens supporting section 20 from the optical section 10 and engaging the lens supporting section 20 again while shifting the position, or the optical section 10 can be detached from the lens supporting section 20 to replace the optical section 10 with a new optical section 10 having a correct power.

Although two engaging holes 54a are formed, one or three or more engaging holes can be formed.

In each of the aforementioned embodiments, the explanation was directed to the case in which the urging member is formed into a curved shape or a loop shape or the case in which the anterior capsule supporting portion, the posterior capsule supporting portion and the connection supporting portion are provided. However, as long as the urging member can expand the anterior capsule Sf and the posterior capsule Sb near the equator Se of the lens capsule S in the fore-and-aft direction by an urging force, any other shape or structure can be employed.

Further, although both of one end portion 201 and the other end portion 202 of the lens supporting section 20 are positioned on a linear line m1 passing the center O of the optical section 10, both of one end portion 201 and the other end portion 202 can be positioned on any other portions as long as they are positioned on opposite sides across the center O of the optical section 10.

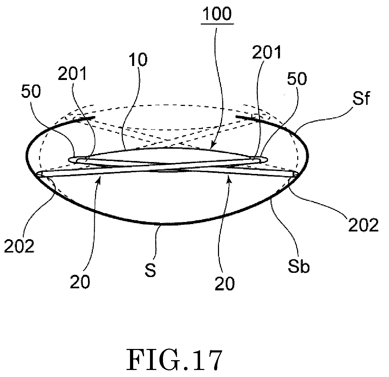

Further, although the aforementioned accommodative intraocular lens is provided with the urging member, as shown in Fig. 17, it can be configured such that no urging member is provided and the other end portion 202 of the lens supporting section 20 is directly engaged with the equator Se of the lens capsule S or therearound.

Further, in this embodiment, although the one end portion or the connecting portion 52, 53 or 54 of the lens supporting section 20 is provided with engaging grooves 53a or engaging holes 54a, such structure can be realized on the other end portion of the the lens supporting section 20.

Although embodiments of the present invention were explained above, the present invention is not limited to the illustrated embodiments. Various corrections or modifications can be applied to the illustrated embodiments within the same scope of the present invention or the equivalent range thereof.

## Claims

1. An accommodative intraocular lens to be inserted into a lens capsule from which contents were removed, comprising:
an optical section made of a lens; and
a plurality of lens supporting sections formed on a peripheral portion of the optical section,
wherein the lens supporting section includes one end portion connected to the peripheral portion of the optical section in a movable manner, and the other end portion to be engaged with an equator of the lens capsule or a lens capsule extension device, the one end portion and the other end portion being positioned on opposite sides across a center of the optical section,
wherein the one end portion extends while branching on both sides of the optical section so as to respectively extend along a periphery of the optical section and then connected with each other to thereby form a loop shape in a manner as to surround the periphery of the optical section as a whole, and
wherein in accordance with movements of the lens capsule or the lens capsule extension device caused by focusing of an eye, the other end portions of the lens supporting sections move in an approaching/departing direction, moving the one end portions of the lens supporting sections in the fore-and-aft direction, which causes movements of the optical section in the fore-and-aft direction in accordance with the movements of the one end portions.

2. The accommodative intraocular lens as recited in claim 1, wherein the one end portion of the lens supporting section is connected to the peripheral portion of the optical section via a connecting portion in a movable manner.

3. The accommodative intraocular lens as recited in claim 2, wherein the one end portion of the lens supporting section or the connecting portion is provided with a slit extending in a circumferential direction of the optical section.

4. The accommodative intraocular lens as recited in claim 2, wherein the connecting portion is provided with one or a plurality of engaging grooves extending in a circumferential direction of the optical section on a rear surface or a front surface of the connecting portion.

5. The accommodative intraocular lens as recited in claim 2, wherein the connecting portion is provided with one or a plurality of engaging holes extending in a circumferential direction of the optical section on a side surface of the connecting portion so that the one end portion of the lens supporting section is engaged with the engaging hole in a movable manner.

6. The accommodative intraocular lens as recited in claim 1, wherein the lens supporting section includes an urging member for urging an anterior capsule and a posterior capsule of the lens capsule in a manner as to distance the anterior capsule and the posterior capsule in the fore-and-aft direction, the urging member being provided at the other end portion of the lens supporting section.

7. The accommodative intraocular lens as recited in claim 6, wherein the urging member is formed into one or a plurality of curved shapes.

8. The accommodative intraocular lens as recited in claim 6, wherein the urging member is formed into one or a plurality of loop shapes.

9. The accommodative intraocular lens as recited in claim 6, wherein the urging member includes an anterior capsule supporting portion supporting the anterior capsule from an inside of the anterior capsule, a posterior capsule supporting portion supporting the posterior capsule from an inside of the posterior capsule, and a connection supporting portion connecting the anterior capsule supporting portion and the posterior capsule supporting portion, and wherein the connection supporting portion urges the anterior capsule supporting portion and the posterior capsule supporting portion in a manner as to distance in the fore-and-aft direction.

10. The accommodative intraocular lens as recited in claim 6, wherein the urging member is provided at its inner side with an engaging member with which the other end portion of the lens supporting section is engaged.

11. The accommodative intraocular lens as recited in claim 10, wherein the engaging member is formed into a plate shape extending in the fore-and-aft direction, wherein one end portion of the engaging member is connected to the one end portion of the urging member and the other end portion of the engaging member is connected to the other end portion of the urging member, and wherein the engaging member is formed in a manner as to bent toward a direction of the equator of the lens capsule.

12. The accommodative intraocular lens as recited in claim 6, wherein a plurality of urging members are provided along a circumference of the lens capsule and adjacent urging members are connected with each other in a circumferential direction.
